Europäisches Patentamt

European Patent Office

Office européen des brevets

(19)

(11) Publication number: **0 278 005**
A1

(12)

# EUROPEAN PATENT APPLICATION

published in accordance with Art. 158(3) EPC

(21) Application number: 87904941.9

(22) Date of filing: 24.07.87

Data of the international application taken as a basis:

(86) International application number:
PCT/JP 87/00543

(87) International publication number:
WO 88/00972 (11.02.88 88/4)

(51) Int. Cl.⁴: **C 12 N 15/00,** C 12 N 1/20
// (C12N1/20, C12R1:19)

(30) Priority: 25.07.86 JP 175140/86

(43) Date of publication of application: 17.08.88
**Bulletin 88/33**

(84) Designated Contracting States: **AT BE CH DE FR GB IT LI LU NL SE**

(71) Applicant: **TAIYO KAGAKU Co., LTD., 9-5, Akahori-Shinmachi, Yokkaishi-shi Mie-ken 510 (JP)**

(72) Inventor: **YAMAZAKI, Nagataka, 4-32, Akahori 2-chome, Yokkaichi-shi Mie-ken 510 (JP)**
Inventor: **YAMAZAKI, Yoshifumi, 8-18, Akahori 1-chome, Yokkaichi-shi Mie-ken 510 (JP)**

(74) Representative: **Barlow, Roy James et al, J.A.KEMP & CO. 14, South Square Gray's Inn, London WC1R 5EU (GB)**

(54) **DNA FOR SHUTTLE VECTOR.**

(57) A DNA usable as vector for animal cells, a process for its preparation, a recombinant plasmid containing the DNA, and transformant bacteria containing the plasmid. The DNA is a part of polyoma virus DNA, has a cleavage point by restriction enzyme BamHI at one end, and has cleavage points by restriction enzyme KpnI at distances of about 0.06 kb and about 3.0 kb or about 3.1 kb from the above-described end, cleavage points by restriction enzyme PvuII at distances of about 0.6 kb and about 1.9 kb and about 2.1 kb, or about 2.0 kb and about 2.2 kb, a cleavage point by restriction enzyme AccI at a distance of about 0.7 kb or about 0.8 kb, a cleavage point by restriction enzyme BglI at a distance of about 0.9 kb or about 1.0 kb, a cleavage point by restriction enzyme EcoRI at a distance of about 2.4 kb or about 2.5 kb, a cleavage point by restriction enzyme HindIII at a distance of about 2.5 kb or about 2.6 kb, and a cleavage point by restriction enzyme HincII at a distance of about 3.8 kb or about 3.9 kb.

BamHI(0)
KpnI (0.06)
HindIII(4.6)
PvuII (0.6)
AccI (0.7)
HincII (4.1)
BglI (0.9)
TYA-I
(5.3 kb)
HincII (3.8)
PvuII(1.9)
KpnI(3.0)
PvuII (2.1)
HindIII
(2.5)
EcoRI (2.4)

00278005

DESCRIPTION

DNAs for use in shuttle vectors

## Technical Field

This invention relates to DNAs for use in vectors for animal cells. Particularly, The invention relates to DNAs which may be cloned into a plasmid inherent in procaryotic cells such as E.coli and B.subtilis or in lower eucaryotic cells such as yeast so as to construct a recombinant plasmid which may in turn function as a shuttle vector with an autonomous growing capability in both these cells and animal cells. This recombinant vector, when introduced into procaryotic or eucaryotic cells with an integrated exogenous gene in interest, may autonomously replicate in an extrachromosome of both procaryotic and eucaryotic cells and produce the exogenous gene in the eucaryotic cells.

## Background Art

The technique has now come to an industrial stage, in which an useful product or commercially important product may be produced by integrating a desired exogenous gene into DNAs of a plasmid or bacteriophage as a vector, transforming E.coli, B.subtilis or the like with the vector and expressing the desired gene therein. By way of examples, insulin and growth hormones may be mentioned as the useful product which were actually prepared using E.coli as a host.

However, it is difficult to produce the desired product, particularly that which has an origin in animal cells by making use of procaryotic cells such as E.coli since such procaryotic cells have no post-translation modification system such as glycosylation nor secretion system out of the cells. On

1

the other hand, animal cells, which have these systems, seem to be potential as a host cell instead of the procaryotic cell. However, a vector suitable for the purpose of a mass production of the useful product by animal cells has not yet developed.

In the present stage, as a possible vector for application in animal cells, there may be mentioned the DNAs of SV40, the modified of SV40 or SVGT-5, polyomavirus, papilomavirus and the like.

However, any one of these DNAs has at least one of the following disadvantages: its integration into a chromosome; a small number of the copies per cell; and its lytic effect on the host cell when the number of the copies is large. Accordingly, they are unsuitable as the vector for the production of the useful gene products.

Consequently, a vector is continually in demand , which has no above disadvantage and may construct a shuttle vector, i.e., a vector which is capable of autonomous replication both in procaryotic cells such as E.coli and animal cells. Because with such shuttle vector it would be possible to easily clone in an economically large scale an exogenous gene integrated therein in procaryotic cells such as E.coli and to express the exogenous gene in animal cells by using the cloned shuttle vector as such.

Though Japanese Patent Application Laying Oepn (KOKAI) NO.60-19492 discloses DNAs which show no lytic activity and exist in the form of a plasmid at a multiple copy, the number of which is still at most about 5,000 per cell. These DNAs are also usable as a shuttle vector.

Disclosure of Invention

Through an intensive research on the vector suitable

2

for the production of the useful gene product using animal cells, the inventors succeeded in obtaining the DNAs defined in claims 1 and 6 by growing the strain F9-28, F9-36 or F9-80 of F9 cells, i.e., teratocarcinoma undifferentiated cells, extracting extrachromosomal DNAs by alkaline denature method (Birnboin and Doly, Nucleic Acids Res., 7; 1513, 1979) and finally purifying the DNAs by equilibrium centrifugation in CsCl-ethidium bromide gradients. The thus-obtained DNAs have the following advantages: (1) they may autonomously replicate in the extrachromosome of animal host cells; (2) they exist in a very high number of copies, that is, about 50,000 copies per cell; (3) the number of copies is amplified by about ten times when the host cells are induced to differentiation; (4) they may express the integrated exogenous gene in the host animal cells while persisting in the extrachromosome of the host cells and autonomously replicating; (5) they may construct a recombinant plasmid with a certain type of the plasmid of procaryotic cells, the recombinant plasmid being in turn served as the above shuttle vector.

Therefore, an object of the present invention is to provide DNAs which are a part of the polyomavirus DNA, characterized in that they have at least a DNA fragment comprising the BamHI site at 0 kb as a standard point, the KpnI sites at about 0.06 kb and about 3.0 kb, the PvuII sites at about 0.6 kb, about 1.9 kb and about 2.1 kb, the AccI site at about 0.7 kb, the BglI site at about 0.9 kb, the EcoRI site at about 2.4 kb, the HindIII site at about 2.5 kb and the HincII site at about 3.8 kb, and to provide DNAs which are a part of the polyomavirus DNA, characterized in that they have at least a DNA fragment comprising the BamHI site at 0 kb as a standard point, the KpnI

3

sites at about 0.06 kb and about 3.1 kb, the PvuII sites at about 0.6 kb, about 2.0 kb and about 2.2 kb, the AccI site at about 0.8 kb, the BglI site at about 1.0 kb, the EcoRI site at about 2.5 kb, the HindIII site at about 2.6 kb and the HincII site at about 3.9 kb

Another object of the present invention is to provide a process for the preparation of the above DNAs.

A further object of the present invention is to provide a recombinant plasmid integrating thereinto the above DNAs and to provide a transformant carrying the recombinant plasmid.

The present invention will be illustrated below in more detail.

Small circular DNAs, TYA-I and TYA-II comprising the DNAs according to the present invention may be obtained from cultured animal cells. For example, the mouse teratocarcinoma F9 cells are plated in each well of five 96-well microtiter plates by a limiting dilution. They are grown in Dulbecco's modified MEM medium supplemented with 10% of fetal calf serum at 37°C for about one week. After the cell suspension is denatured by an alkaline denature solution (0.2N NAOH, 1% sodium dodecyl sulfate (SDS)), the cloned cells are centrifuged at 1,500 r.p.m. for 10 minutes to give DNAs, which are then separated by 0.8% agarose gel electrophoresis according to the differences in molecular size between the DNAs. The cultured cell strain from which only the DNAs of a lower molecular weight (TYA-I) may be obtained is designated as F9-28, and the cultured cell strain from which only the DNAs of a higher molecular weight (TYA-II) may be obtained is designated as F9-36. Furthermore, the cultured strain carrying both TYA-I and TYA-II is designated as F9-80.

4

According to agarose gel electrophoresis, the molecular sizes of TYA-I and TYA-II are determined to be about 5.3 kb and about 5.4 kb, respectively. Such difference in molecular size may be attributable to the difference in length between the PvuII-AccI fragment of TYA-I and that of TYA-II, that is, the latter is about two times longer than the former.

Restriction endonuclease analyses indicate that TYA-I has the BamHI site at 0 kb as a standard point, the KpnI sites at about 0.06 kb and about 3.0 kb, the PvuII sites at about 0.6 kb, about 1.9 kb and about 2.1 kb, the AccI site at about 0.7 kb, the BglI site at about 0.9 kb, the EcoRI site at about 2.4 kb, the HindIII sites at about 2.5 kb and about 4.6 kb and the HincII sites at about 3.8 kb and about 4.1 kb, and that TYA-II has the BamHI site at 0 kb as a standard point, the KpnI sites at about 0.06 kb and about 3.1 kb, the PvuII sites at about 0.6 kb, about 2.0 kb and about 2.2 kb, the AccI site at about 0.8 kb, the BglI site at about 1.0 kb, the EcoRI site at about 2.5 kb, the HindIII sites at about 2.6 kb and about 4.7 kb and the HincII sites at about 3.9 kb and about 4.2 kb.

In comparison of TYA-I and -II with the DNAs of polyomavirus, it is found that their EcoRI, KpnI and HincII sites are very similarly located to those of polyomavirus. But there are three PvuII sites in TYA-I and -II whereas the polyomavirus DNAs has four the same sites.

Japanese Patent Application Laying Open No.60-19492 discloses the plasmid vectors for animal cells, LFI and LFII, which are similar in structure to polyomavirus. These two vectors have the SalI site but no BglI site while TYA-I and -II have the BglI site but no SalI site.

Southern blot analysis (Southern E., Mol. Biol., 98, 503, 1975) has revealed that both TYA-I and -II are present at about 50,000 copies per cell.

High molecular extraction of DNAs from F9-28, -36 or -80 by SDS-proteinkinase K procedure (Blin N. and D. W. Stafford, Nucleic Acids Res., 3, 2303, 1976) is performed, followed by separation of the DNAs by agarose gel electrophoresis according to the difference in molecular weight. The following Southern blot analyses demonstrate that TYA-I and -II exist extrachromosomally as a small circular DNA with a capacity of autonomous replication.

TYA-I or -II may be cloned into plasmid vector pUC19 so as to construct a recombinant vector which can grow in E. coli. That is to say, TYA-I or -II is cleaved by means of BamHI and converted into a linear DNA molecule. Vector pUC19 is similarly cleaved with the same enzyme. These two cleved DNA fragments are then ligated with each other to give the recombinant plasmid which is designated as pTYA-I or pTYA-II. The recombinant plasmid is then introduced into E.coli strain JM83 by transformation. The transformant thus obtained, JM83-I or JM83-II, is grown in a large scale, collected and extracted to give DNAs, from which a large amount of extrachromosomal DNAs may be separated by equilibrium centrifugation in CsCl-ethidium bromide gradients.

Brief Description of Drawings

FIG.1 is a restriction endonuclease cleavage map for TYA-I.

FIG.2 is a restriction endonuclease cleavage map for TYA-II.

FIG.3 shows a recombinant plasmid pTYAE.

6

FIG.4 shows a recombinant plasmid pF9-CAT.

Industrial Applicability

The DNAs according to the present invention may be cloned into the plasmid of E.coli so as to construct a recombinant plasmid which can function as the shuttle vector.

For example, the BamHI-HincII DNA fragment of TYA-I or -II and the BamHI-PvuII DNA fragment of E.coli plasmid pBR 322 are ligated with each other to give the recombinant plasmid named pTYAE. E.coli is transformed with pTYAE and cultured. Then, dam-methylated pTYAE DNAs, which have been cloned in the transformed E.coli as a host, are collected and used for transfection into  mouse LTk$^-$ cells. Southern blot analysis of the extrachromosomal DNAs in mouse LTk$^-$ cells shows no dam methylation of the duplicated pTYAE DNAs. This result suggests that pTYAE is capable of autonomous replication even in the extrachromosome of mouse LTk$^-$ cells since a dam-methylase is not present in animal cells. Consequently, the DNAs according to the present invention may be cloned into the E.coli plasmid to construct the recombinant plasmid usable as a shuttle vector between E.coli and animal cells.

Another usefulness of the DNAs according to the invention is derived from their capacity to function as a vector which may integrate a gene for the commercially important product and exist extrachromosomally in animal cells and then express the gene.

To confirm the capacity, the epression of a gene coding for a chloramphenicol acetyltransferase (CAT gene) in F9 cell strains which are deficient of the same gene is examined using a recombinant plasmid from the DNAs accordiong to the invention and

7

the fragment of the plasmid comprising the CAT gene.

For example, the DNAs according to the invention, that is, the BamHI-HincII DNA fragment of TYA-I or TYA-II, the BamHI-PvuII DNA fragment of pBR 322 and the PvuII-BamHI DNA fragment of $pSV_2CAT$ which is a plasmid having the CAT gene are ligated with one another to yield the recombinant plasmid named pF9CAT. After a two-day incubation of F9Tk⁻ cells transfected with pF9CAT, the cells are extracted. The cell extract thus obtained is subjected to CAT assay. The result shows the production of acetyl chloramphenicol in the transfected F9Tk⁻ cells.

Therefore, a rapid improvement in the productivity of the commercially important product using animal cells as a host may be expected by making use of the DNAs according to the present invention in the shuttle vector.

Furthermore, upon in vitro differentiation by the addition of some chemicals to undifferentiated cells carrying the DNAs according to the invention, the copy number of which may be amplified. For example, the chemical is added to F9-28 strains, which are then cultured for differentiation. The amount of the extrachromosomal DNAs extracted from the strains is tested by Southern blot analysis every other day. It is consequently recognized that on Day 4 in the differentiation the copy number of the DNAs has been amplified about ten times as many as that of the undifferentiated cells , that is, from about 50,000 copies to 500,000 copies per cell.

As the above chemicals, any which is conventionally used to induce differentiation may be used, dibutyl cAMP and retinoic acid being preferred.

8

Accordingly, it may be easily derived that the productivity of the useful product is further improved by the induction to differentiation of F9 cells, which is effected after they are transfected with the recombinant plasmid containing the exogenous gene encoding the useful product and are cultured.

## Best Mode for Carrying Out the Invention

## EXAMPLE 1:

## 1. Preparation of TYA-I and TYA-II

(1) About $5 \times 10^8$ of the cultured cell strain F9-28, a clone of mouse F9 cells were collected and suspended in 2 ml of GTE buffer (25mM Tri-HCl, pH8.0, 50mM glucose and 1mM EDTA). To this 4 ml of an alkaline denaturing solution (0.2N NaOH, 1% SDS) was added. The resulting mixture was well mixed and allowed to stand at 0°C for 5 minutes. After an addition of potassium acetate (5M, 3 ml) cooled on ice, the mixture was allowed to stand in ice for 10 minutes for neutralization and cetrifuged at 30,000 r.p.m. for 30 minutes. The supernatant was extracted with an equal volume of phenol and three volumes of ethylether successively. To the aqueous layer 2.5 volumes of ethanol was added so as to precipitate DNAs, which were then dissolved in 3.5 ml of TE buffer (10mM Tris-HCl, pH7,5, 1mM EDTA). After an addition of 4.15 g of CsCl and 0.5 ml of ethidium bromide (5 mg/l), the solution was centrifuged at 50,000 r.p.m. for 15 hours. The band obtained was separately collected and deprived of ethidium bromide by the addition of butyl alcohol to give about 10 ug of DNAs, which were designated as TYA-I.

(2) In a similar manner as described above, the DNAs designated as TYA-II were obtained from the cultured cell strain F9-36, a clone of mouse F9 cells.

## 2  Determination of molecular size

To each fraction of the DNAs obtained in the above, 1 ul of BamHI (10 u/ul), 2 ul of BamHI buffer (0.5M NaOH, 0.1M Tris-HCl, pH 7.5, 0.1M $MgCl_2$, 10mM DTT) diluted ten times and 16 ul of water were added and the mixture was kept at 37°C for 60 minutes. Determination of the molecular size was performed by agarose gel electrophoresis (0.8% agarose, 0.5ug/ml ethidium bromide), in which the HindIII fragments of lambda DNA were used as a marker. The results obtained showed that TYA-I and TYA-II have the molecular sizes of about 5.3 kb and about 5.4 kb, respectively.

## 3  Preparation of restriction endonuclease cleavage map

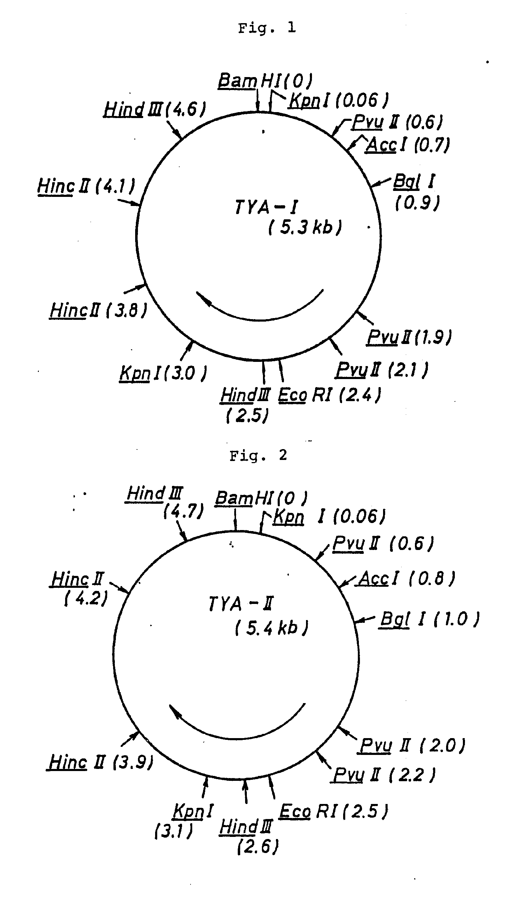

One ug each of TYA-I, TYA-II and the DNAs of the wild type polyomavirus strain A2 was digested with BamHI, EcoRI, BglI, HindIII, KpnI, PvuII, SalI, AccI and HincII of 10 units each. The resulting fragments were separated on a 0.8 - 1.5% agarose gel electrophoresis. By using the fragment of the DNAs of A2 strain as a marker, the restriction endonuclease cleavage maps were obtained, which are shown in FIG. 1 and FIG. 2.

## 4  Determination of copy number

F9-28 strains of about $1 \times 10^7$ cells were washed three times with PBS (phosphate buffer saline) and suspended in 10 ml of NET buffer (100mM NaCl, 10mM EDTA, 10mM Tris-HCl, pH7.5). To this 10% SDS solution was added to a final concentration of 0.5%. Then, proteinkinase K was added to a final concentration of 100 ug/ml and the resulting mixture was sufficiently mixed and incubated at 37°C for 10 hours. An equal volume of phenol solution (being saturated in 0.1M Tris-HCl, pH7.5) was added to the reaction mixture. After a vigorous mixing the resulting

10

mixture was centrifuged so as to separate an aqueous layer from a phenol layer. The extraction of the aqueous layer with phenol was carried out until white impurities disappeared from the interface between phenol and water. After phenol remaining in the water layer was removed by the extraction with ether, the chromosomal DNA and TYA-I were co-precipitated with ethanol. RNA was digested with ribonuclease A. The content of TYA-I was determined by absorbance at 260 nm. Southern blot analysis ( Southern E., J. Mol. Biol. 98, 503, 1975) was performed, in which the length of the chromosomal DNA was supposed to be $3 \times 10^6$ kb. Using as a standard the DNAs of TYA-I clones whose amounts corresponded to $10^4$ per cell, $10^5$ per cell and $10^6$ per cell, respectively, the copy number of TYA-I per cell was determined to be about 50,000.

The same procedure performed on F9-36 strains showed the presence of TYA-II at about 50,000 copies per cell.

## 5  Demonstration of no integaration of TYA-I or -II into the chromosome of a host cell

The chromosomal DNAs and extrachromosomal DNAs were extracted from F9-28 strains by SDS-proteinkinase K procedure (Blin N. and W. Stafford, Nucleic Acids Res., 3, 2303, 1976) and separated on agarose gel electrophoresis. The BamHI-HincII fragment of TYA-I was purified and labelled with $^{32}$P by nick translation (Maniatis, Proc. Natl. Acad. Sci. U.S.A. 72:114, 1975). The labelled fragment, which was complementary to TYA-I was served as a probe in Southern blot analysis. This technique showed no hybridization between the probe and the chromosomal DNA fragments, indicating that TYA-I was not integrated into the chromosome of F9-28 strains.

The same results were obtained on TYA-II in F9-36 strains and on TYA-I and -II in F9-80 strains.

**EXAMPLE 2:**

**Demonstration of autonomous replication in E coli of a recombinant plasmid with the plasmid of procaryotic cells**

TYA-II was cleaved with <u>Bam</u>HI and converted into a linear form. The linearized TYA-II was then inserted at the <u>Bam</u>HI site of plasmid vector pUC19 having an ampicillin-resistance gene. The recombinant plasmid thus obtained was designated as pTYA-II, which was introduced into <u>E.coli</u> strain JM83. The resulting transformant was named JM83-II.

One ug of TYA-II was digested with <u>Bam</u>HI by incubating in a reaction mixture containing 5 units of the enzyme at 37°C for one hour. pUC19 was similarly treated with the same enzyme. Both DNAs were purified by extracting with phenol and precipitating with ethanol. For ligation, these purified DNAs were mixed in the presence of 2.5 units of $T_4$ ligase in ligation buffer (50mM Tris-HCl, pH7.4, 10mM $MgCl_2$, 10mM PTT, 1mM ATP) and kept at 16°C for one hour.

For transformation of JM83, the above ligated DNAs were added to 150 ul of JM83 which had been treated with CT solution (50mM $CaCl_2$, 10mM Tris-HCl, pH8.0) at 0°C for 20 minutes. This mixture was kept at 0°C for 2 minutes, heated to 37°C for 3 minutes and then cooled again at 0°C for 5 minutes. One ml of L-broth (polypeptone 1%, yeast extract 0.5%, NaCl 0.5%) was added to the mixutre. After a cultivation in the mixture at 37°C for 60 minutes, the transformants were plated on agar plates containing L-broth with ampicillin of 50 ug/ml and then cultured at 37°C overnight. Colonies resistant against ampicillin were selected and single colonies were grown in 1.5 ml of L-broth. Each

12

cultured colony was centrifuged at 10,000r.p.m. for 10 minutes. The pellet was washed with STE-1 buffer (8% sucrose, 50mM Tris-HCl, pH8.0, 50mM EDTA) and dissolved in STET (the STE-1 buffer containing 5% of Triton X-100). To this 1 ml of a lysozyme solution (10 mg/ml) was added. After an incubation at a room temperature, the lysate solution was heated at 100°C for 1.5 minutes and centrifuged at 30,000r.p.m. for 30 minutes. The supernatant was mixed with an equal volume of isopropyl alcohol and allowed to stand at a room temperature for 30 minutes. The precipitated DNAs were dissolved in 3.5 ml of TE buffer. After an addition of 4.15g of CsCl and 0.5 ml of ethidium bromide (5 mg/ml) the TE buffer solution was centrifuged at 50,000r.p.m. for 15 hours to yield about 1mg of DNAs. The digestion of the resulting DNAs with BamHI showed the presence of TYA-II within pUC19. This result proved that TYA-II and the E.coli plasmid can construct the recombinant plasmid which is capable of autonomous replication in E.coli.

The result was the same in the case of TYA-I.

EXAMPLE 3:

Demonstration of extrachromosomal auto-replication of the recombinant plasmid between the DNAs according to the invention and the plasmid of procaryotic cells

TYA-I (3ug) was treated with BamHI and HincII solution (50mM NaCl, 10mM Tris-HCl, pH7.5, 10 units of BamHI, 10 units of HincII) at 37°C for 10 minutes. The BamHI-HincII fragment (about 3.8 kb) was purified by a 0.8% agarose gel electrophoresis. This DNA fragment (0.1ug) and 0.1 ug of the BamHI-PvuII fragment of pBR322 comprising an ampicillin resistance gene (about 2.3kb) were ligated with each other in the presence of 2.5 units of $T_4$

13

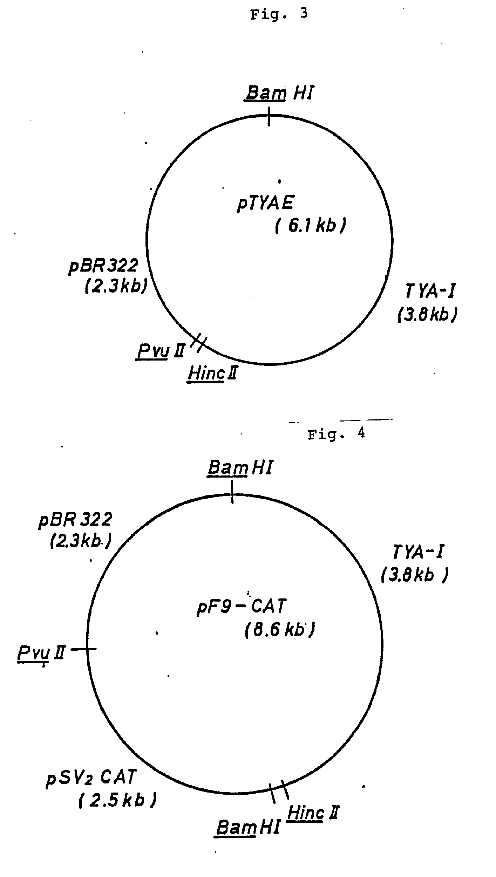

ligase at 16°C for one hour. The resulting recombinant plasmid was named pTYAE (FIG.3).

pTYAE was introduced into E.coli HB101 treated with CT solution for transformation. Colonies habouring pTYAE were selected on agar plates containing ampicillin (50 ug/ml). LTk⁻ mouse cells (about 1 x 10^6) were mixed with 1 ug of pTYAE and transfection buffer (0.1% dextrose, 140mM NaCl, 5M $CaCl_2$, 1mM $Na_2HPO_4$, 125mM $CaCl_2$, 20mM HEPES buffer, pH7.05). The mixture was incubated in a $CO_2$ incubator at 37°C for 48 hours. From this transfected cells the extrachromosomal DNAs were extracted by Hirt's method (Hirt B., J. Mol. Biol., 26, 365, 1967). The DNAs extract was treated with MboI and DpnI, separated by agarose gel electrophoresis and then subjected to Southern blot analysis using as a probe the DNAs complementary to an enhancer region of TYA-I. The DNAs obtained from LTk⁻ cells were found to be sensitive to MboI but not to DpnI. This results showed that DNAs without methyl groups were amplified in LTk⁻ cells which have no dam methylase as a result that TYA-I which had dam-methylated on adenines in E.coli was replicated in LTk⁻ cells. Accordingly, the DNAs of the present invention may be cloned into the plasmid of E.coli so as to construct the shuttle vector which can extrachromosomally replicate both in E.coli and in eucaryotic cells.

The same is true for TYA-II.

EXAMPLE 4:

Demonstration of expression of the gene coding for a commercially important product and integrated into the DNAs of the present invention

CAT gene was integrated into the recombinant plasmid

14

pTYAE prepared in EXAMPLE 3 and successfully expressed.

TYA-I (3ug) was treated with the BamHI-HincII solution (50mM NaCl, 10mM Tris-HCl, pH7.5, 10 units of BamHI, 10 units of HincII) at 37°C for 10 minutes. The BamHI-HincII DNA fragment (about 3.8 kb) was purified by 0.8% agarose gel electrophoresis. In the same manner, the BamHI-PvuII DNA fragment of pBR322 (about 2.3kb) and the BamHI-PvuII DNA fragment of pSV$_2$CAT were purified. The BamHI-PvuII DNA fragment of pSV$_2$CAT was treated with Klenow reaction solution (50mM Tris-HCl, pH7.2, 10mM MgSO$_4$, 0.1mM dithiothreitol(DTT), 1mM disodium d-adenosine-5'-triphosphate (dATP), 1mM sodium d-guanosine-5'-triphosphate (dGTP), 1mM sodium d-cytidine-5'-triphosphate (dCTP), 1mM sodium d-thymidine-5'-triphosphate (dTTP), 1 unit Klenow fragment of DNA polymerase I of E.coli) at 22°C for 30 minutes. For ligation 0.1 ug of the Klenow-treated fragment was mixed with 0.1 ug of the BamHI-PvuII DNA fragment of pBR322 and 0.1 ug of BamHI-HincII DNA fragment of TYA-I in the same ligation buffer as in EXAMPLE 2. On adding 2.5 units of T$_4$ DNA ligase the mixture was incubated at 16°C for one hour, producing a novel recombinant plasmid which was designated as pF9-CAT (FIG.4). Similarly, the recombinant plasmid was prepared by replacing the DNA fragment of TYA-I by the corresponding DNA fragment of the wild type polyomavirus. This plasmid was designated as pPy-CAT. E.coli HB101 treated with CT solution was transformed with these recombinant plasmids and clones carrying the same plasmids were selected on agar plates containing 50 ug/ml of ampicillin.

F9Tk$^-$ mouse cells (F9 cells carrying no TYA-I or -II) were mixed with 20 ug of pF9-CAT or pPy-CAT and transfection buffer (0.1% dextrose, 140mM NaCl, 5M CaCl$_2$, 1mM Na$_2$HPO$_4$, 125mM

$CaCl_2$, 20mM HEPES buffer, pH7.05). The mixture was incubated in a $CO_2$ incubator at 37°C for 5 hours. After adding 3 ml of Dulbecco's modified MEM medium containing 10% of fetal calf serum and 30% glycerol, the mixture was incubated for 30 seconds, washed with the medium and then incubated again in the $CO_2$ incubator at 37°C for 2 days.

The transfected cells were collected and suspended in 100mM Tris-HCl (pH7.8). The cell suspension was subjected to three cycles of freezing-thawing method followed by ultrasonication for 5 minutes and centrifugation at 15,000 r.p.m. for 10 minutes. The resulting supernatant (100ul) was mixed with 40 ul of 4mM acetyl-CoA and 1.5 ul of $^{14}C$ chloramphenicol. The mixture was incubated at 37°C for one hour and extracted with ethyl acetate. After an evaporation of ethyl acetate under vacuum, chloramphenicol and acetyl derivatives were separated by ascending in thin layer chromatography. Autoradiography showed a radio activity of acetylchloramphenicol in the extract from the cells transfected with pF9-CAT, which indicates that the DNAs of the invention may integrate the exogenous gene (CAT gene), which is then expressed in mouse F9 cells.

The same result was obtained by using TYA-II.

EXAMPLE 5:

Demonstration of amplification of the copy number of TYAs by induction to differentiation

F9-28 strains were suspended in a fresh Dulbecco's modified MEM medium containing 10% of fetal calf serum. For differentiation $10^{-3}$M dibutyl cyclic AMP and $10^{-7}$ retinoic acid were added to the cell suspension and the mixture was incubated in a $CO_2$ incubator at 37°C. The cells were collected on 0, 2, 4,

16

5, 6 and 8 days after the above addition and the total cellular DNAs of the induced cells on each day were respectively extracted by SDS-proteinkinase K method. As described in EXAMPLE 1, 4., the contnent of the chromosomal DNAs of each extract was determined and an aliquot of each extract containing 10 ug of the chromosomal DNAs was sampled. With this procedure the comparison of the copy number of TYA-I per a certain number of the cells was possible. Southern blot analysis using as a probe the DNA fragment complementary to TYA-I indicated that the amount of the DNAs detected by the probe on Day 4 was ten times as much as that before the induction. Accordingly, the copy number of TYA-I may be amplified by the induction to differentiation of the host cells.

The same results were obtained in the experiment using TYA-II.

CLAIMS

1. DNAs which are a part of the polyomavirus DNA, characterized in that they have at least a DNA fragment comprising the BamHI site at 0 kb as a standard point, the KpnI sites at about 0.06 kb and about 3.0 kb, the PvuII sites at about 0.6 kb, about 1.9 kb and about 2.1 kb, the AccI site at about 0.7 kb, the BglI site at about 0.9 kb, the EcoRI site at about 2.4 kb, the HindIII site at about 2.5 kb and the HincII site at about 3.8 kb.

2. DNAs according to claim 1, wherein the DNAs further comprise the HincII site at about 4.1 kb and the HindIII site at about 4.6 kb.

3. DNAs according to claim 1 or 2, wherein the molecular size is approximately 5.3 kb.

4. DNAs according to claim 1, 2 or 3, wherein the DNAs are circular ones obtained from mouse F9 cells.

5. DNAs according to claim 4, wherein the mouse F9 cells are cultured cell strains F9-28 or F9-80 and the DNAs are designated as TYA-I.

6. DNAs which are a part of the polyomavirus DNA, characterized in that they have at least a DNA fragment comprising the BamHI site at 0 kb as a standard point, the KpnI sites at about 0.06 kb and about 3.1 kb, the PvuII sites at about 0.6 kb, about 2.0 kb and about 2.2 kb, the AccI site at about 0.8 kb, the BglI site at about 1.0 kb, the EcoRI site at about 2.5 kb, the HindIII site at about 2.6 kb and the HincII site at about 3.9 kb.

7. DNAs according to claim 6, wherein the DNAs further

18

comprise the <u>Hinc</u>II site at about 4.2 kb and the <u>Hind</u>III site at about 4.7 kb.

8. DNAs according to calim 6 or 7, wherein the molecular size is approximately 5.4 kb.

9. DNAs according to claim 6, 7 or 8, wherein the DNAs are circular ones obtained from mouse F9 cells.

10. DNAs according to claim 9, wherein the mouse F9 cells are cultured cell strains F9-36 or F9-80 and the DNAs are designated as TYA-II.

11. A process for the preparation of DNAs which comprises:

    culturing mouse F9 cells;

    lysing the cultured cells; and,

    separating the polyomavirus DNAs.

12. A process according to claim 11, wherein the mouse F9 cells are cultured cell strains F9-28.

13. A process according to claim 11, wherein the mouse F9 cells are cultured cell strains F9-36.

14. A process according to claim 11, wherein the mouse F9 cells are cultured cell strains F9-80.

15. A process according to claim 12 or 14, wherein the DNAs are TYA-I.

16. A process according to claim 13 or 14, wherein the DNAs are TYA-II.

17. A recombinant plasmid pTYA-I.

18. A recombinant plasmid pTYA-II.

19. A transformant JM83-I carrying the recombinant plasmid pTYA-I.

20. A transformant JM83-II carrying the recombinant plasmid pTTYA-II.

Fig. 1

Fig. 2

00278005

Fig. 3

Fig. 4

# INTERNATIONAL SEARCH REPORT

00273005

International Application No  PCT/JP87/00543

**I. CLASSIFICATION OF SUBJECT MATTER** (if several classification symbols apply, indicate all) ³

According to International Patent Classification (IPC) or to both National Classification and IPC

Int.Cl⁴   C12N15/00, 1/20 // (C12N1/20, C12R1:19)

**II. FIELDS SEARCHED**

Minimum Documentation Searched ⁷

| Classification System | Classification Symbols |
|---|---|
| IPC | C12N15/00 |

Documentation Searched other than Minimum Documentation
to the Extent that such Documents are Included in the Fields Searched ⁵

**III. DOCUMENTS CONSIDERED TO BE RELEVANT** ¹⁴

| Category * | Citation of Document, ¹⁶ with indication, where appropriate, of the relevant passages ¹⁷ | Relevant to Claim No. ¹⁸ |
|---|---|---|
| A | JP, A, 60-19492 (Taisho Pharmaceutical Co., Ltd.) 31 January 1985 (31. 01. 85) & EP, A2, 135278 | 1-20 |
| A | JP, A, 62-134084 (New York University) 17 June 1987 (17. 06. 87) (Family: none) | 1-20 |
| A | JP, A, 57-122097 (F. Hoffmann-La Roche & Co., A.G.) 29 July 1982 (29. 07. 82) & EP, A2, 54223 | 1-20 |

* Special categories of cited documents: ¹⁵
"A"  document defining the general state of the art which is not considered to be of particular relevance
"E"  earlier document but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance: the claimed invention cannot be considered novel or cannot be considered to involve an inventive step
"Y"  document of particular relevance: the claimed invention cannot be considered to involve an inventive step when the document is combined with one or more other such documents, such combination being obvious to a person skilled in the art
"&"  document member of the same patent family

**IV. CERTIFICATION**

| Date of the Actual Completion of the International Search ² | Date of Mailing of this International Search Report ² |
|---|---|
| September 24, 1987 (24. 09. 87) | October 5, 1987 (05. 10. 87) |

| International Searching Authority ¹ | Signature of Authorized Officer ²⁰ |
|---|---|
| Japanese Patent Office | |

Form PCT/ISA/210 (second sheet) (October 1977)